# EUROPÄISCHE PATENTSCHRIFT

(11) **EP 0 988 022 B1**
(45) Veröffentlichungstag und Bekanntmachung des Hinweises auf die Patenterteilung: **21.09.2005**
(21) Anmeldenummer: 98941388.5
(22) Anmeldetag: 21.07.1998
(51) Int. Cl.: A61K 7/13

(54) **HAARFÄRBEMITTEL**
HAIR DYE
PRODUITS DE COLORATION CAPILLAIRES

(30) Priorität: 25.07.1997 DE 19732016
(43) Veröffentlichungstag der Anmeldung: 29.03.2000
(73) Patentinhaber: Henkel Kommanditgesellschaft auf Aktien, 40191 Düsseldorf (DE)
(72) Erfinder: ROSE, David, D-40723 Hilden (DE); MEINIGKE, Bernd, D-51381 Leverkusen (DE); HÖFFKES, Horst, D-40595 Düsseldorf (DE); MÖLLER, Hinrich, D-40789 Monheim (DE)
(86) Internationale Anmeldenummer: PCT/EP1998/004544
(87) Internationale Veröffentlichungsnummer: WO 1999/007334

(56) Entgegenhaltungen:
- US-A- 5 474 578

## Beschreibung

Die Erfindung betrifft die Verwendung von Methinfarbstoffen einer spezifischen Struktur zum Färben von Keratinfasern, insbesondere von menschlichen Haaren.

Für das Färben von keratinischen Fasern spielen neben den Oxidationsfarbstoffen, die zu besonders echten, permanenten Haarfärbungen führen, besonders die direktziehenden Haarfarbstoffe eine wichtige Rolle. Diese Farbstoffe erzeugen auf dem Haar semipermanente oder temporäre Färbungen, die eine geringere Stabilität gegen das Haarewaschen aufweisen. Als direktziehende Farbstoffe werden vorwiegend Verbindungen eingesetzt, die zur Gruppe der Nitrobenzolderivate gehören. Diese weisen aber oft nicht die zur Färbung aus wäßriger Flotte erforderliche Wasserlöslichkeit auf. Andere bekannte direktziehende Farbstoffe sind z.B. Anthrachinonfarbstoffe, Indophenole, Triphenylmethanfarbstoffe und kationische Azofarbstoffe.

Die kationischen Farbstoffe haben den Vorteil einer guten Wasserlöslichkeit und eines guten Aufziehvermögens auf die meist negativ geladene Proteinfaser.

Aus WO 95/01772 A1 und WO 95/15144 A1 sind z.B. kationische Azö- und Azomethinfarbstoffe bekannt, die sich auch zur Haarfärbung unter physiologisch annehmbaren Bedingungen der Temperatur und der Verweilzeit eignen. Ein Problem, das bei der Verwendung solcher Farbstoffe zum Haarefärben besteht, ist eine zu starke Anfärbung der Kopfhaut.

Die US-A-5 474 578 offenbart ein Verfahren zur Haarfärbung mit Methinfarbstoffen, wobei die Färbung durch Bekandlung mit Peroxid im alkalischen Milieu wieder rückgängig gemacht werden kann.

Die DE-A-3 210 596 offenbart die Verwendung bestimmter Methinfarbstoffe zum Färben von Baumwolle, Polyamid- und Polyesterfasern, Papier, Leder und zur Herstellung von Tinten.

Es bestand daher die Aufgabe, solche Farbstoffe zu finden, die bei niedriger Temperatur und in kurzer Verweilzeit intensive Farbnuancen ausbilden und die Kopfhaut nicht zu stark anfärben. Die Farbstoffe sollen außerdem eine gute Stabilität gegen Licht, Wärme, Schweiß und Haarwaschmittel sowie gegen die bei der Dauerwellung des Haars verwendeten Chemikalien aufweisen. Schließlich sollen sie auch in toxikologischer und dermatologischer Hinsicht unbedenklich sein. Sie sollten auch eine gute Verträglichkeit mit anderen Farbstoffen und z.B. auch mit Oxidationsfarbstoffen und den in Oxidationsfärbemitteln üblichen Komponenten aufweisen, da man zur Nuancenabwandlung gerne direktziehende Farbstoffe unterschiedlichen Typs und Oxidationsfarbstoffe kombiniert. Daher ist auch eine gute Stabilität gegen Oxidations- und Reduktionsmittel erforderlich.

Es wurde nun gefunden, daß bestimmte Methinfarbstoffe die gestellten Anforderungen in hohem Maße erfüllen:

Gegenstand der Erfindung ist daher die Verwendung von Methinfarbstoffen der Formel (I) 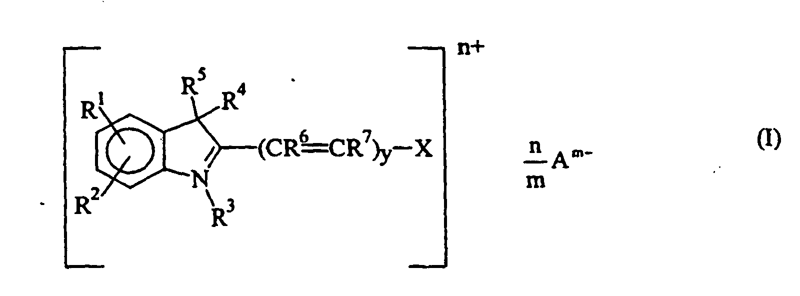 zum Färben von Keratinfasern, insbesondere von menschlichen Haaren.

In der Formel (I) stehen R¹ und R² unabhängig voneinander für Wasserstoff, eine C₁- bis C₄-Alkylgruppe, eine C₁- bis C₄-Alkoxygruppe, eine Hydroxygruppe, eine Aminogruppe oder eine Mono- bzw. Di-(C₁- bis C₄-alkyl)-aminogruppe,
R³ steht für Wasserstoff, eine C₁- bis C₄-Alkylgruppe oder eine C₂- bis C₄-Hydroxyalkylgruppe,
R⁴ bis R⁷ stehen unabhängig voneinander für Wasserstoff oder eine C₁- bis C₄-Alkylgruppe, X steht für eine Gruppe der Formeln (II) bis (IV) 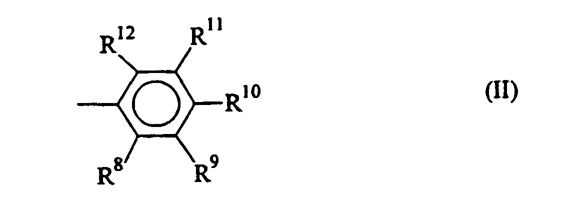 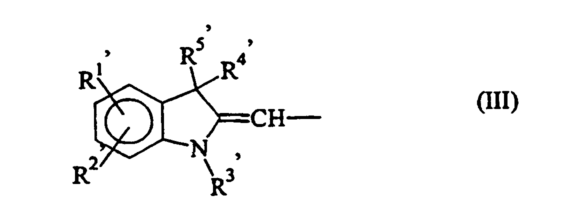 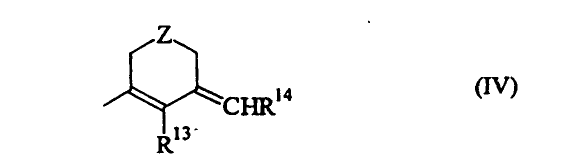 und y ist eine ganze Zahl von 1 bis 3, n die Zahl der positiven Ladungen im Kation und A ist ein m-wertiges physiologisch verträgliches Anion.

In Substituenten gemäß der Formel (II) können R⁸ und R¹² unabhängig voneinander stehen für Wasserstoff, ein Halogenatom, eine Hydroxygruppe, eine C₁- bis C₄-Alkoxygruppe oder eine C₂- bis C₄-Hydroxyalkylgruppe,
R⁹ und R¹¹ für stehen Wasserstoff oder bilden jeweils gemeinsam mit R'° eine Methylendioxygruppe,
R¹⁰ steht weiterhin für eine C₁- bis C₄-Alkoxygruppe, eine C₂- bis C₄-Hydroxyalkylgruppe, oder eine Gruppe der Formel (V)

-NR¹⁵R¹⁶ (V)

wobei R¹⁵ und R¹⁶ unabhängig voneinander stehen für eine C₁- bis C₄-Alkylgruppe, eine C₂- bis C₄-Hydroxyalkylgruppe oder gemeinsam mit dem Stickstoffatom einen Pyrrolidin-. Piperidin- oder Piperazinring bilden,
jeweils 2 benachbarte Substituenten R⁸ bis R¹² einen ankondensierten Benzolring bilden oder der Substituent gemäß Formel (II) ein 2,3,6,7-Tetrahydro-1H,5H-pyrido[3,2,1-ij]chinolinrest ist.

In Verbindungen der Formel (I), in denen X für einen Substituenten der Formel (III) steht, entspricht der Substituent R^{1'} dem Substituenten R¹ oder einer der anderen genannten Bedeutungen von R¹. Entsprechendes gilt für R^{2'} und R¹, R^{3'} und R³, R^{4'} und R⁴ sowie für R^{5'} und R⁵.

In Verbindungen der Formel (I), in denen X für einen Substituenten der Formel (IV) steht, steht R¹³ für Wasserstoff, ein Halogenatom, eine C₁- bis C₄-Alkylgruppe oder eine C₁- bis C₄-Alkoxygruppe, R¹⁴ für einen Phenyl- oder Naphthylrest, der durch C₁- bis C₄-Alkylgruppen, C₁- bis C₄-Alkoxygruppen, eine Hydroxygruppe, eine Carboxygruppe oder eine Mono- beziehungsweise Di-(C₁- bis C₄-alkyl)-aminogruppe, wobei die Alkylreste mit dem Phenyl- oder Naphthylrest auch einen oder zwei ankondensierte Heterocyclen oder mit dem Aminstickstoff einen Heterocyclus bilden können, substituiert sind, sowie für Pyridyl-, Chinolyl-, Furyl-, Thienylreste oder die Gruppe (III) und Z ist eine direkte Bindung, eine Methylengruppe, eine Dimethylmethylengruppe oder eine Ethylengruppe.

Sofern es sich bei den erfindungsgemäßen Substanzen um Amino-Verbindungen handelt, lassen sich diese in Abhängigkeit des pH-Werts durch Protonierung in die entsprechenden quaternären Verbindungen überführen. Alle Aussagen dieser Schrift und demgemäß der beanspruchte Schutzbereich beziehen sich daher sowohl auf die in freier Form vorliegenden Verbindungen gemäß Formel (I) als auch auf deren protonierte Derivate.

Unter keratinischen Fasern sind erfindungsgemäß Pelze, Wolle, Federn und insbesondere menschliche Haare zu verstehen.

Beispiele für die als Substituenten in den erfindungsgemäßen Verbindungen genannten C₁-bis C₄-Alkylgruppen, sind die Gruppen Methyl, Ethyl, Propyl, Isopropyl und Butyl, Ethyl- und Methylgruppen sind bevorzugte Alkylgruppen. Ferner können C₁- bis C₄-Alkoxygruppen als Substituenten auftreten, beispielsweise eine Methoxy- oder eine Ethoxygruppe. Erfindungsgemäße Beispiele für eine C₁- bis C₄-Hydroxyalkylgruppe, sind eine Hydroxymethyl-, eine 2-Hydroxyethyl-, eine 3-Hydroxypropyl- oder eine 4-Hydroxybutylgruppe. Als Substituenten geeignete Halogenatome sind beispielsweise ein F-, ein Cl- oder ein Br-Atom.

Als Anion A^{m-} eignen sich z.B. Chlorid, Bromid, Jodid, Sulfat, Perchlorat, Tetrafluorborat, Tetraphenylborat und Tetrachlorozinkat.

Ein besonderer Vorteil der erfindungsgemäß zu verwendenden Methinfarbstoffe der Formel (I) besteht in einem hohen Egalisierungsvermögen, d.h., daß der Farbstoff auf Haarschaft und Haarspitze etwa gleich stark aufzieht, so daß die stärker strapazierten Haarspitzen nicht intensiver angefärbt werden als der glatten Haarschaft am Haaransatz.

Die Farbstoffe der Formel (I) sind teilweise literaturbekannt, solche, die nicht bekannt sind, lassen sich nach literaturbekannten Herstellverfahren gewinnen. Die Herstellung zweier literaturunbekannter Farbstoffe der Formel (I) ist im Beispielteil näher beschrieben.

Bevorzugt geeignete Farbstoffe der Formel (I) sind solche, bei denen R³ eine Methylgruppe ist.

Weiterhin bevorzugte Farbstoffe der Formel (I) im Sinne der vorliegenden Erfindung, sind solche, bei denen R⁴ und R⁵ Methylgruppen sind.

Es ist nicht erforderlich, daß der Farbstoff als einheitliche Verbindung vorliegt, es kann auch ein Gemisch verschiedener Farbstoffe der Formel (I) als Farbstoff verwendet werden. Die Verbindungen der Formel (I) liefern intensive, licht- und reibechte Färbungen im Bereich gelber, roter und violetter Nuancen, wobei im neutralen und alkalischen pH-Bereich etwas dunklere Nuancen erhalten werden als im sauren pH-Bereich.

Ein weiterer Gegenstand der Erfindung sind Haarfärbemittel, die Methinfarbstoffe der Formel (I) gemäß Anspruch 1 in einer Menge von 0.01 bis 10 Gew.-%, bezogen auf das gesamte Haarfärbemittel, in einem zum Färben geeigneten Träger enthalten.

Besonders bevorzugt im Sinne der vorliegenden Erfindung sind Färbemittel in einem wäßrigen Träger oder in Pulverform.

Gemäß einer ersten bevorzugten Ausführungsform der Erfindung handelt es sich um solche Mittel, die nur eine vorübergehende Färbung der Faser bewirken sollen. Solche Mittel werden häufig als Tönungsmittel bezeichnet. Diese Ausführungsform umfaßt beispielsweise auch solche Haarbehandlungsmittel, mit denen die Haare nicht nur vorübergehend gefärbt, sondern auch zu einer bestimmten Frisur gestylt werden sollen. In diesem Falle spricht man von Tönungsfestigern.

Da solche Mittel üblicherweise ohne die Zuhilfenahme von oxidierenden Komponenten, insbesondere Wasserstoffperoxid, formuliert werden, sind die erfindungsgemäßen Mittel gemäß dieser Ausführungsform frei von den üblichen Oxidationsfarbstoffvorprodukten vom Kuppler- und Entwicklertyp.

Wenngleich die Verbindungen gemäß Formel (I) auch als alleinige Farbstoffkomponente eingesetzt werden können, so enthalten die Mittel gemäß dieser Ausführungsförm bevorzugt noch mindestens einen weiteren Farbstoff vom Typ der Direktzieher. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin. Pikraminsäure, 2-Amino-6-chloro-4-nitrophenol 4-Ethylamino-3-nitrobenzoesäure und 2-Chloro-6-ethylamino-1-hydroxy-4-nitrobenzol.

Unter direktziehenden Farbstoffen werden erfindungsgemäß auch Naturfarbstoffe wie beispielsweise Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel verstanden.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Die weiteren Direktzieher sind in den erfindungsgemäßen Mitteln bevorzugt in Mengen von jeweils 0,01 bis 20 Gew.-% bezogen auf das gesamte Mittel enthalten.

Gemäß einer zweiten bevorzugten Ausführungsform handelt es sich bei den beanspruchten Mitteln um Haarfärbemittel für die dauerhafte Färbung der Haare. Diese Mittel können mindestens ein Oxidationsfarbstoffvorprodukt vom Typ der Entwickler enthalten. Diese, in der Regel farblosen, Verbindungen reagieren unter der Einwirkung von Oxidationsmitteln oder von Luftsauerstoff, gegebenenfalls mit Hilfe spezieller Enzyme oder Metallionen als Katalysator, mit sich unter der Ausbildung der gewünschten Farbstoffe. Insbesondere zur Ausbildung natürlicher Haarfarbtöne werden aber in der Regel Kombinationen von mehreren Entwicklerkomponenten eingesetzt. Weiterhin wird in der Regel mindestens ein sogenannter Kuppler eingesetzt, der unter dem Einfluß von Oxidationsmitteln mit den Entwicklerkomponenten reagieren, was zu neuen Farben bzw. einer Nuancierung der Farbe führt. Erfindungsgemäß kann sowohl eine Kupplerkomponente als auch mehrere Kupplerkomponenten alleine oder in Kombination mit einer oder mehreren Entwicklerkomponenten eingesetzt werden.

Als Entwicklerkomponenten werden üblicherweise primäre aromatische Amine mit weiteren, in para- oder ortho-Position befindlichen, freien oder substituierten Hydroxy- oder Aminogruppen, Diaminopyridinderivate, heterocyclische Hydrazone. 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triamino-pyrimidin, 2,4-Dihydroxy-5,6-diaminopyrimidin, 2-Dimethylamino-4,5,6-triamino-pyrimidin, 2-Hydroxyethylaminomethyl-4-amino-phenol, 4,4'-Diaminodiphenyl-amin, 4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propa-nol, 4-Amino-2-(2-hydroxyethoxy)-phenol sowie 4,5-Diaminopyrazol-Derivate nach EP 0 740 741 bzw. WO 94/08970 wie z.B. 4,5-Diamino-1-(2'-hydroxyethyl)-pyrazol.

Besonders bevorzugte Entwickler im Sinne der vorliegenden Erfindung sind p-Aminophenol, 1-(2'-Hydroxyethyl)-2,5-diaminobenzol, 2,4,5,6-Tetraaminopyrimidin, 2-Hydroxy-4,5,6-triaminopyrimidin, 4-Hydroxy-2,5,6-triaminopyrimidin, 4,5-Diamino-1-(2-hydroxyethyl)-pyrazol, p-Toluylendiamin, 2-Aminomethyl-4-amino-phenol, N,N-Bis-(2-hydroxy-ethyl)-p-phenylendiamin, p-Phenylendiamin, 3-Methyl-p-aminophenol und Bis-(2-hydroxy-5-aminophenyl)-methan.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, Pyrogallol, 1,5-, 2,7- und 1,7-Dihydroxynaphthalin, o-Aminophenol, 5-Amino-2-Methylphenol, m-Aminophenol. Resorcin, Resorcinmonomethylether, m-Phenylendiamin. 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2.5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin.

Besonders bevorzugte Kupplerkomponenten sind 1-Naphthol, 1,5-, 2,7- und 1,7-Dihydroxynaphthalin, Resorcin, 2,5-Dimethylresorcin, 2,6-Dihydroxy-3,4-diaminopyridin, m-Aminophenol, 5-Amino-2-methylphenol, 3-Methylsulfonyl-amino-2-methyl-anilin, 3-Amino-2-methylamino-6-methoxy-pyridin, 2,6-Dimethyl-3-amino-phenol, 2,4-Diaminophenoxyethanol, 6-Methyl-1,2,3,4-tetrahydro-chinoxalin, 2-Methyl-4-chlor-5-aminophenol, 3,4-Methylendioxyanilin, 2-Methyl-resorcin, 5-Methylresorcin, 4-Chlorresorcin, 3,4-Methylendioxyphenol, 2-Amino-3-hydroxypyridin und 2-Chlor-6-methyl-3-aminophenol.

Erfindungsgemäß können solche Entwickler- und Kupplerkomponenten, die für die Ausbildung der Färbung keine Oxidationsmittel außer Luftsauerstoff benötigen, bevorzugt sein.

Die erfindungsgemäßen Haarfärbemittel enthalten sowohl die Entwicklerkomponenten als auch die Kupplerkomponenten bevorzugt in einer Menge von 0,005 bis 20 Gew.-%, jeweils bezogen auf das Färbemittel ohne die Oxidationsmittelzubereitung. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem MolVerhältnis von 1 : 0,5 bis 1 : 2 enthalten sein können.

Die erfindungsgemäßen Mittel dieser Ausführungsform können außer den Verbindungen gemäß Formel (I), den Entwickler- und/oder Kupplerkomponenten gewünschtenfalls zur Nuancierung noch weitere direktziehende Farbstoffe enthalten. Es sei an dieser Stelle auf die oben angegebene Auflistung verwiesen.

Es ist nicht erforderlich, daß die Farbstoffe und Farbstoffvorprodukte jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsuntemehmen für Arzneimittel, Reformwaren und Körperpflegemittel e.V., Mannheim, Bezug genommen.

Gemäß einer bevorzugten Ausführungsform enthalten die erfindungsgemäßen Mittel, insbesondere, wenn sie noch konditionierende oder festigende Eigenschaften aufweisen sollten, weiterhin anionische, nichtionogene oder insbesondere kationische Polymere.

Als konditionierende Wirkstoffe geeignete kationische Polymere enthalten innerhalb des Polymergerüstes kationische Gruppen. Diese Gruppen können Teil der Polymerkette sein, sie können sich aber auch in Seitenketten befinden, die über Zwischenglieder mit einer Hauptkette verbunden sind.-Übliche kationische Gruppen enthalten quartäre Stickstoff- oder Phosphoratome. Gruppen mit quartären Stickstoffatomen sind dabei bevorzugt. Die quartären Stickstoffatome können dabei sowohl 4 unterschiedliche oder z.T. gleiche Substituenten tragen, als auch Teil eines Ringsystems sein. Bevorzugte kationische Gruppen sind Ammonium- und Imidazoliumgruppen.
Beispiele für solche Polymere sind:
- quaternierte Cellulose-Derivate, wie sie unter den Bezeichnungen Celquat® und Polymer JR® im Handel erhältlich sind. Die Verbindungen Celquat® H 100, Celquat L 200 und Polymer JR®400 sind bevorzugte quaternierte Cellulose-Derivate.
- quaternierte Guar-Derivate, wie sie unter den Bezeichnungen Cosmedia Guar® und Jaguar® im Handel erhältlich sind. Bevorzugte Guar-Derivate sind beispielsweise Cosmedia Guar® C-261 und Jaguar® C 13-S.
- Copolymere des Vinylpyrrolidons mit quaternierten Derivaten des Dialkylaminoalkylacrylats- und -methacrylats, wie beispielsweise mit Diethylsulfat quaternierte Vinylpyrrolidon-Dimethylaminoalkylmethacrylat-Copolymere sowie das Vinylpyrrolidon-Methacrylamidopropyltrimethylammoniumchlorid-Copolymere. Solche Verbindungen sind unter den Bezeichnungen Gafquat®734, Gafquat®755 bzw. Gafquat® HS100 im Handel erhältlich.
- Copolymerisate des Vinylpyrrolidons mit Vinylimidazoliummethochlorid, wie sie unter der Bezeichnung Luviquat® angeboten werden.
- Polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere.
- Kationisch derivatisierte Silikonöle, wie beispielsweise die im Handel erhältlichen Produkte Q2-7224 (Hersteller: Dow Corning; ein stabilisiertes Trimethylsilylamodimethicon), Dow Coming 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).
- Chitosan und dessen Derivate

Bevorzugt sind die kationischen Polymeren in den erfindungsgemäßen Mitteln in Mengen von 0,1 - 5 Gew.-%, bezogen auf die gesamte Zubereitung, enthalten.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) vertrieben werden.
- Schellack
- Polyvinylpyrrolidone, wie sie beispielsweise unter der Bezeichnung Luviskol® (BASF) vertrieben werden.

Beispiele für geeignete anionische Polymere sind:
- Copolymere der Acrylsäure und/oder Methacrylsäure oder deren Ester mit C₁₀₋₃₀-Alkylacrylaten, wie sie beispielsweise unter der Bezeichnung Pemulen® vertrieben werden.
- Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere. Verbindungen dieser Art sind unter den Markenbezeichnungen Resyn® (NATIONAL STARCH), Luviset® (BASF) und Gafset® (GAF) im Handel; die Produkte Luviset®CA-66 und Luviset®CAP können bevorzugt sein.
- Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymere.
- Acrylsäure/Ethylacrylat/N-tert.Butylacrylamid-Terpolymere, die beispielsweise unter der Bezeichnung Ultrahold® strong (BASF) vertrieben werden, sowie Methacrylsäure/Ethylacrylat/t-Butylacrylat-Terpolymere, die unter der Bezeichnung Luvimer®100P (BASF) vertrieben werden.

Die Verwendung von anionischen, nichtionogenen oder kationischen Polymeren kann in solchen Haarfärbemitteln bevorzugt sein, die frei von Oxidationsfarbstoffvorprodukten sind.

Zur Herstellung der erfindungsgemäßen Färbemittel werden die direktziehenden Farbstoffe und gegebenenfalls die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist.
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylamrnonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmineln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlond. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex® vertriebenen Methyl-hydroxyalkyldialkoyloxyalkyl-atnmoniummethosulfate sowie die unter dem Warenzeichen Dehyquart® vertriebenen Produkte wie Dehyquart AU-46.

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen. Alkalimetallhydroxiden oder Alkalimetallalkoholaten als Katalysatoren erhält. Eingeengte Homologenverteilungen werden dagegen erhalten, wenn beispielsweise Hydrotalcite. Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside liegen in den erfindungsgemäßen Mitteln bevorzugt in Mengen von 1-20, insbesondere etwa 6-10 Gew.-%, jeweils bezogen auf das gesamte Mittel, vor, wenn es sich bei dem erfindungsgemäßen Mittel um ein Färbeshampoo handelt. Bei Färbelotionen oder Färbegelen sind dagegen Mengen von 1-10, insbesondere von 2-4 Gew.-%, jeweils bezogen auf das gesamte Mittel, bevorzugt.

Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäwealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Es ist prinzipiell möglich, die erfindungsgemäßen Mittel so zu formulieren, daß sie entweder auf dem Haar verbleiben oder wieder aus dem Haar ausgespült werden. Gemäß einer bevorzugten Ausführungsform werden die erfindungsgemäßen Mittel so formuliert, daß sie auf dem Haar verbleiben können. Dies ist insbesondere bei sogenannten Tönungsmitteln der Fall, aber auch bei Mitteln, die außerdem eine festigende Funktion ausüben sollen.

Gegenstand der Anmeldung ist auch die Verwendung eines erfindungsgemäßen Mittels zum Färben und Tönen von keratinischen Fasern, insbesondere menschlichen Haaren.

Die nachfolgenden Beispiele sollen den Gegenstand der Erfindung näher erläutern:

### Beispiele

### I. Farbstoffe

Es wurden die folgenden Methinfarbstoffe hergestellt:

### Beispiel 1

R³, R⁴, R⁵ = CH₃; R¹⁰ = OCH₃; R¹² = Cl; R¹, R², R⁶, R⁷, R⁸, R⁹, R¹¹ = H;
y=1; n = 1, m = 2, A = ZnCl₄.

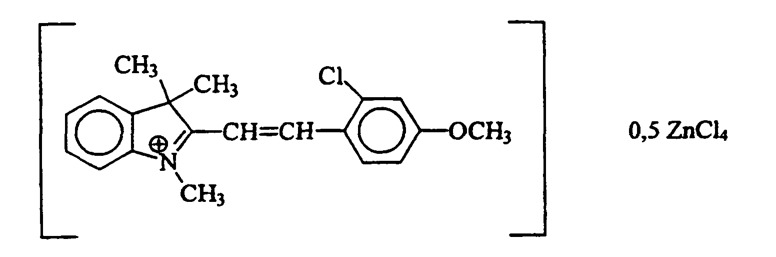

### Herstellung:

0,05 Mol 2-Methylen-1,3,3-trimethylindolin wurde in 24 ml Eisessig bei 20°C gelöst. 0,05 Mol 2-Chlor-4-methoxybenzaldehyd wurde zur Lösung gegeben und diese dann 4 Stunden auf 80°C erwärmt. Nach dem Abkühlen auf 20°C wurde mit 500 ml Wasser verdünnt und eine Lösung von 7,5g (0,055mol) ZnCl₂ in 50 ml konz. Salzsäure zugegeben. Das ausgefällte Produkt wurde abfiltriert und bei ca. 60°C im Vakuum getrocknet. Es wurden rote Kristalle erhalten.

### Beispiel 2

R³, R⁴, R⁵ = CH₃; R¹⁰, R¹¹ = O-CH₂-O-; R¹, R² R⁶, R⁷, R⁸, R⁹, R¹² = H;
y=1; n = 1, m = 2, A = ZnCl₄.

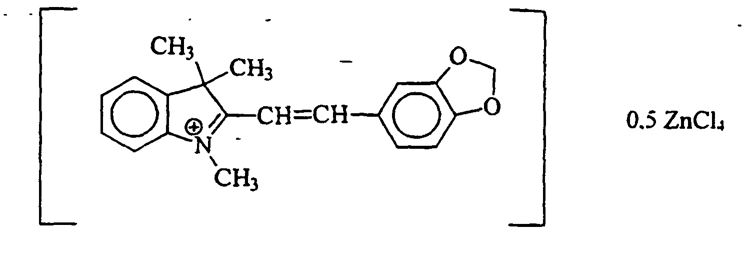

Die Herstellung erfolgte analog Beispiel 1, wobei anstelle von 2-Chlor-4-methoxybenzaldehyd der 3,4-Methylendioxybenzaldehyd (Piperonal) eingesetzt wurde.

### Beispiel 3

R³, R⁴, R⁵, R¹⁵, R¹⁶ = CH₃; R¹⁰ = NR¹⁵R¹⁶; R¹, R², R⁶, R⁷, R⁸, R⁹, R¹¹, R¹² = H;
y=1; n = 2,
a) m = 1, A = J
b) m = 2, A = ZnCl₄

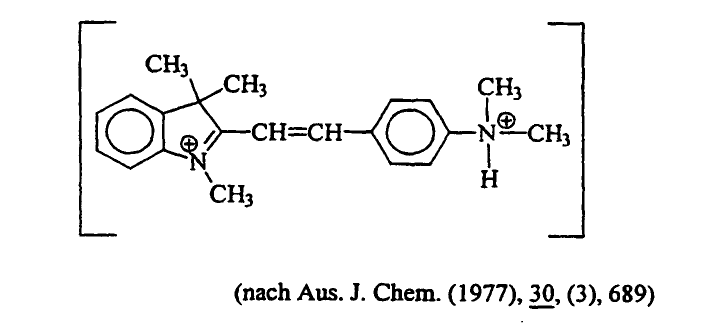

### Beispiel 4

R³, R⁴, R⁵, = CH₃; R¹⁵, R¹⁶ = C₂H₅; R¹⁰ = NR¹⁵R¹⁶; R¹, R², R⁶, R⁷, R⁸, R⁹, R¹¹, R¹² = H;
y=1; n = 2,
a) m = 1, A = Cl
b) m = 2, A = ZnCl₄.

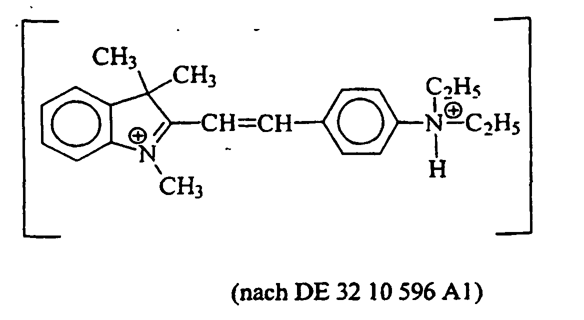

### Beispiel 5

R³, R⁴, R⁵ = CH₃; R'° = Pyrrolidino; R¹, R², R⁶, R⁷, R⁸, R⁹, R¹¹, R¹² = H;
y = 1; n = 1,
a) m = 1, A = J
b) m = 2, A = ZnCl₄.

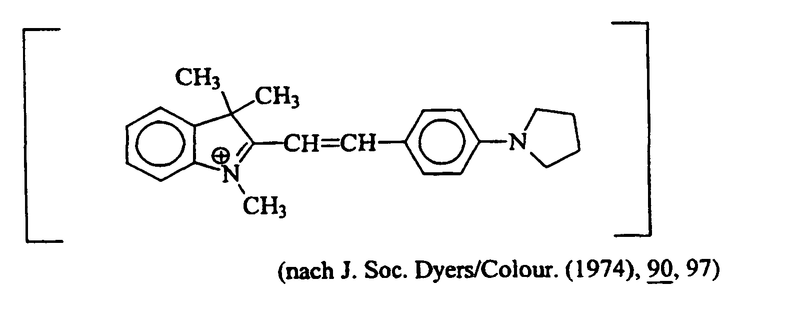

### Beispiel 6

R³, R⁴, R⁵, R¹⁵, R¹⁶ = CH₃; R¹⁰ = NR¹⁵R¹⁶; R¹² = OCH₃; R¹, R², R⁶, R⁷, R⁸, R⁹, R¹¹ = H;
y=1; n = 2, m = 2, A = ZnCl₄.

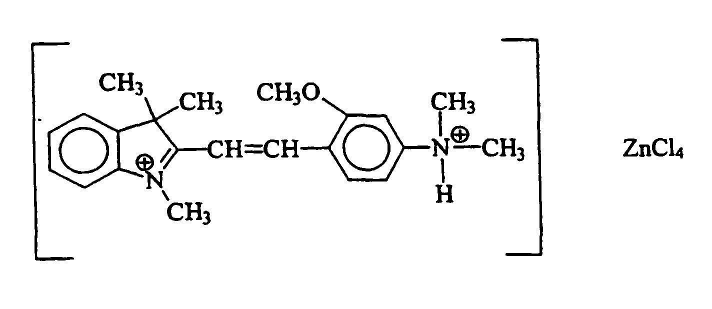

Die Herstellung erfolgte analog Beispiel 1 unter Einsatz von 2-Methoxy-4-dimethylaminobenzaldehyd. Es wurden grüne Kristalle (FP : 95 -100°C) erhalten.

### Beispiel 7

R³, R⁴, R⁵ = CH₃; X ist ein 2,3,6,7-Tetrahydro-1H,5H-pyrido[3,2,1-ij]chinolinrest: R¹, R²,
R⁶, R⁷, R⁸, R¹² = H; y=1; n=1,
a) m = 1, A = ClO₄
b) m = 2, A = ZnCl₄.

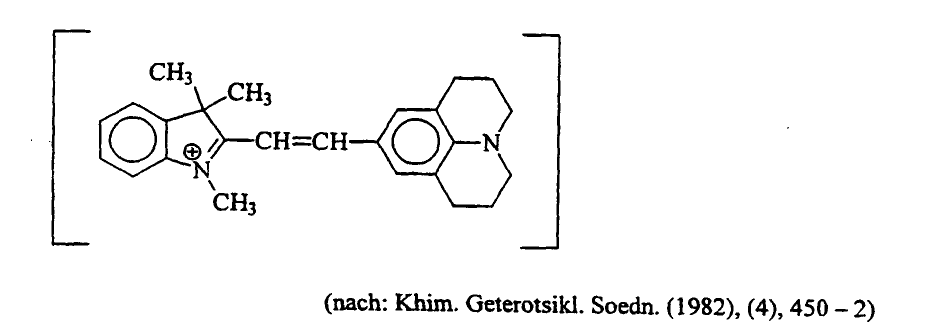

### Beispiel 8

R³, R⁴, R⁵, R^{3'}, R^{4'}, R^{5'} = CH₃; X= Rest der Formel (III); R¹, R², R^{1'}, R^{2'},R⁶, R⁷ = H;
y=2; n = 1, m = 1, A = J.

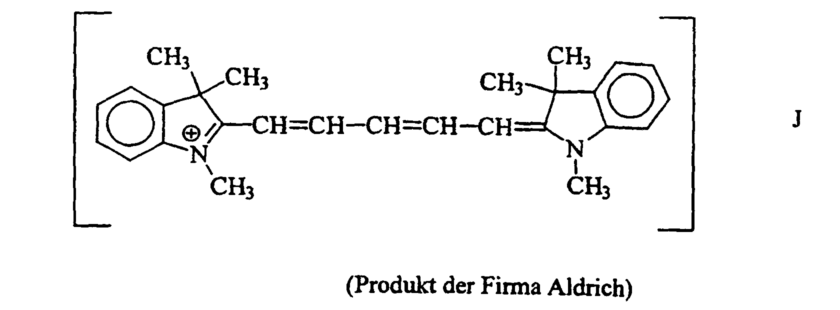

### Beispiel 9

R³, R⁴, R⁵, R¹⁵, R¹⁶ = CH₃; R¹⁰ = NR¹⁵R¹⁶; R¹, R², R⁶, R⁷, R⁸, R⁹, R¹¹, R¹²= H;
y=2; n = 1, m = 1, A = p-Toluolsulfonat.

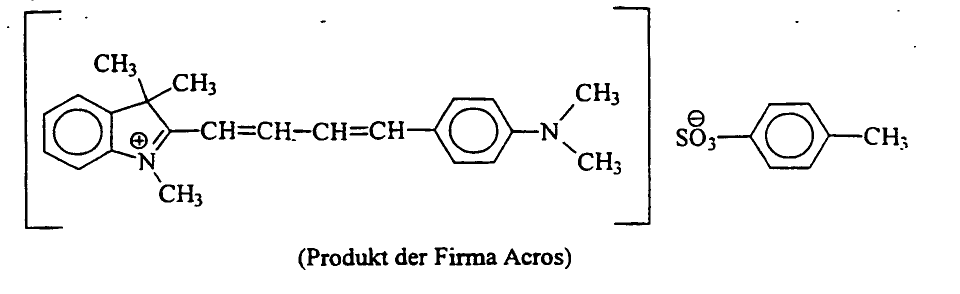

### Beispiel 10

R³, R⁴, R⁵ = CH₃; R¹⁰ = NR¹⁵R¹⁶; R¹, R², R⁶, R⁷, R⁸, R⁹, R¹¹= H; R¹² = OH; R¹⁵, R¹⁶=C₂H₅;
y=1; n = 2, m = 2, A = ZnCl₄.

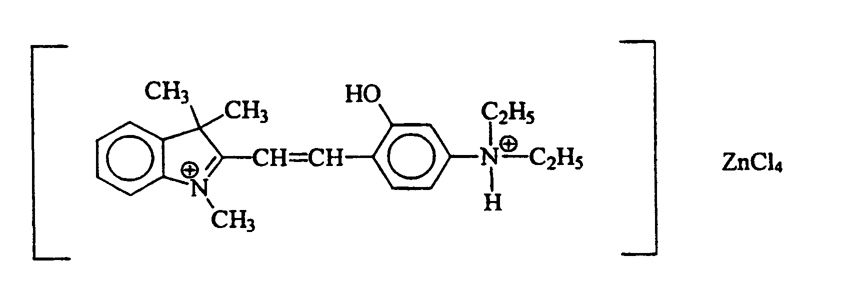

### Beispiel 11

R³, R⁴, R⁵ = CH₃; R¹⁰, R¹² = OCH₃; R¹, R², R⁶, R⁷, R⁸, R⁹, R¹¹ = H;
y=1; n = 1, m = 2, A = ZnCl₄.

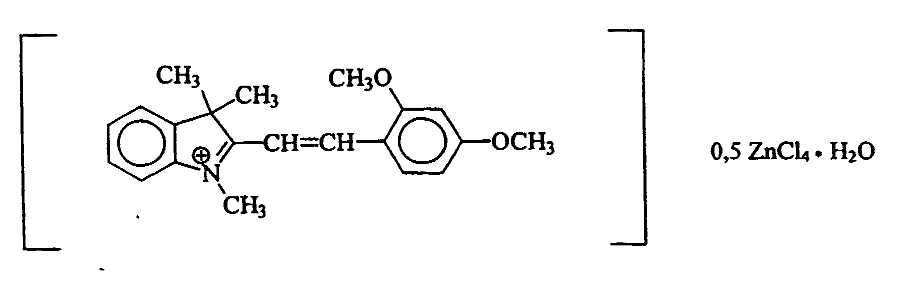

### Beispiel 12

R³, R⁴, R⁵, R¹⁵, R¹⁶ = CH₃; R¹⁰ = NR¹⁵R¹⁶; R¹, R², R⁶, R⁷, R¹¹, R¹² = H; R⁸ bildet mit R⁹ einen ankondensierten Benzolring; y =1; n = 2, m = 2, A = ZnCl₄.

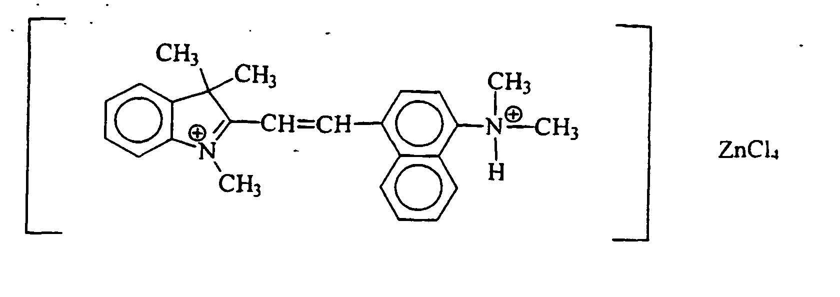

### Haarfärbeversuche

Es wurden Haarfärbecremes der folgenden Zusammensetzung hergestellt:
- Anionische Basis

| | |
|---|---|
| Fettalkohol C₁₂ - C₁₈ | 10 g |
| Fettalkohol C₁₂-C₁₈ + 2EO-sulfat, Na-Salz (28 %ig) | 25 g |
| Wasser | 55 g |
| Methinfarbstoff (Beispiel 1 - 9) | 1 g |
| Ammoniumsulfat | 1 g |
| Wasser und NH₃ oder NH₄HSO₄ zur pH-Wert-Einstellung | 8 g |

Die Bestandteile wurden der Reihe nach miteinander vermischt. Nach Zugabe des Methinfarbstoffs wurde mit konz. Ammoniak-Lösung oder mit NH₄HSO₄-Lösung der pH-Wert auf den gewünschten Wert (5, 7 oder 9) eingestellt und mit Wasser auf 100 g aufgefüllt.
- Nichtionische Basis
Teilmischung A

| | |
|---|---|
| Hydrenol® D¹ | 6g |
| Lorol® techn.² | 2g |
| Cremophor® A25³ | 2g |

| | |
|---|---|
| ¹ C₁₆-C₁₈ Fettalkohol (INCI-Bezeichnung: Cetearylalkohol) (HENKEL) | |
| ² C₁₂- bis C₁₈-Fettalkohol (INCI-Bezeichnung: Coconut alcohol) (HENKEL) | |
| ³ Talgalkohol mit 25-EO (INCI-Bezeichnung: Ceteareth-25) (BASF) | |

Teilmischung B

| | |
|---|---|
| Ammoniumsulfat | 1g |
| Methinfarbstoff | 1g |
| Ammoniaklösung, 25%ig | q.s. |

Die Teilmischung A wurde aufgeschmolzen, mit 60g Wasser (80°C) vermischt und unter starkem Rühren emulgiert. Danach wurde die Emulsion unter schwachem Rühren abgekühlt.
Die Teilmischung B wurde in 10g Wasser (50°C) gelöst. Anschließend wurde die Teilmischung B zur Emulsion A gegeben und der pH-Wert mit einer 25%igen Ammoniaklösung wie gewünscht eingestellt. Es wurde bis zum Erreichen der Raumtemperatur weitergerührt.

Die Färbecremes wurde auf 5 cm lange Strähnen standardisierten, zu 90% ergrauten, aber nicht besonders vorbehandelten Menschenhaars aufgetragen und dort 30 Minuten bei 27°C belassen. Nach Beendigung des Färbeprozesses wurde das Haar gespült, mit einem üblichen Shampoo ausgewaschen und anschließend getrocknet.

Das Ergebnis der Färbeversuche ist der folgenden Tabelle zu entnehmen:

**Tabelle**

| Farbstoff | Basis | pH - Wert | Nuance des gefärbten Haars |
|---|---|---|---|
| Beispiel 1 | Anionisch | 9 | dunkelmagenta |
| Beispiel 1 | Nichtionisch | 7 | cerise |
| Beispiel 2 | Anionisch | 5 | gelb |
| Beispiel 2 | Nichtionisch | 7 | gelborange |
| Beispiel 3a | Anionisch | 7 | tiefmagenta |
| Beispiel 3b | Nichtionisch | 7 | graumagenta |
| Beispiel 4a | Anionisch | 7 | tiefmagenta |
| Beispiel 4a | Nichtionisch | 7 | graumagenta |
| Beispiel 5a | Anionisch | 7 | orchideenpurpur |
| Beispiel 5b | Nichtionisch | 7 | orchideenpurpur |
| Beispiel 6 | Anionisch | 7 | violettrot |
| Beispiel 6 | Nichtionisch | 7 | magenta |
| Beispiel 7a | Anionisch | 5 - 9 | tiefviolett |
| Beispiel 7b | Nichtionisch | 7 | tiefviolett |
| Beispiel 8 | Nichtionisch | 9 | chinablau |
| Beispiel 9 | Nichtionisch | 7 | tiefblau |
| Beispiel 10 | Nichtionisch | 7 | magenta |
| Beispiel 11 | Nichtionisch | 7 | orangegelb |
| Beispiel 12 | Nichtionisch | 5 | dunkelblau |

## Patentansprüche

1. Verwendung von Methinfarbstoffen der Formel (I) 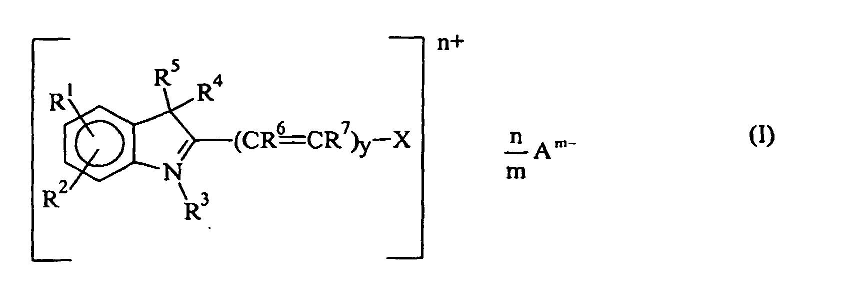 in der R¹ und R² unabhängig voneinander stehen für Wasserstoff, eine C₁- bis C₄-Alkylgruppe, eine C₁- bis C₄-Alkoxygruppe, eine Hydroxygruppe, eine Aminogruppe oder eine Mono- bzw. Di-(C₁- bis C₄-alkyl)-aminogruppe,
R³ steht für Wasserstoff, eine C₁- bis C₄-Alkylgruppe oder eine C₂- bis C₄-Hydroxyalkylgruppe,
R⁴ bis R⁷ stehen unabhängig voneinander für Wasserstoff oder eine C₁- bis C₄-Alkylgruppe und
X steht für eine Gruppe der Formeln (II) bis (IV) 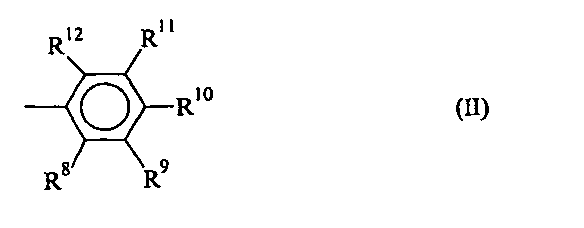 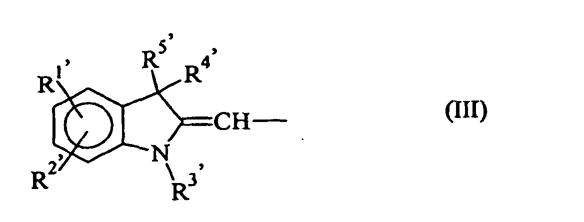 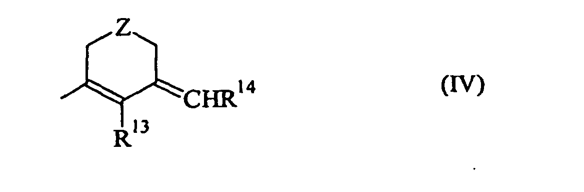 wobei R^{1'} bis R^{5'} für eine der entsprechend unter R¹ bis R⁵ genannten Gruppen steht,
R⁸ und R¹² unabhängig voneinander stehen für Wasserstoff, ein Halogenatom, eine Hydroxygruppe, eine C₁- bis C₄-Alkoxygruppe oder eine C₂- bis C₄-Hydroxyalkylgruppe,
R⁹ und R¹¹ für Wasserstoff stehen oder jeweils gemeinsam mit R¹⁰ eine Methylendioxygruppe bilden,
R'° weiterhin steht für eine C₁- bis C₄-Alkoxygruppe, eine C₂- bis C₄-Hydroxyalkylgruppe,
eine Gruppe der Formel (V)
-NR¹⁵R¹⁶ (V)
wobei R¹⁵ und R¹⁶ unabhängig voneinander stehen für eine C₁- bis C₄-Alkylgruppe, eine C₂- bis C₄-Hydroxyalkylgruppe oder gemeinsam mit dem Stickstoffatom einen Pyrrolidin-, Piperidin- oder Piperazinring bilden,
zwei benachbarte Substituenten R⁸ bis R¹² jeweils gemeinsam einen ankondensierten Benzolring bilden
oder der Substituent gemäß Formel (II) ein 2,3,6,7-Tetrahydro-1H,5H-pyrido[3,2,1-ij]chinolinrest ist,
R¹³ steht für Wasserstoff, ein Halogenatom, eine C₁- bis C₄-Alkylgruppe oder eine C₁-bis C₄-Alkoxygruppe,
R¹⁴ steht für einen Phenyl- oder Naphthylrest, der durch C₁- bis C₄-Alkylgruppen, C₁-bis C₄-Alkoxygruppen, eine Hydroxygruppe, eine Carboxygruppe oder eine Monobeziehungsweise Di-(C₁- bis C₄-alkyl)-aminogruppe, wobei die Alkylreste mit dem Phenyl- oder Naphthylrest auch einen oder zwei ankondensierte Heterocyclen oder mit dem Aminstickstoff einen Heterocyclus bilden können, substituiert sind, sowie für Pyridyl-, Chinolyl-, Furyl-, Thienylreste oder den Rest (III),
Z eine direkte Bindung, eine Methylengruppe, eine Dimethylmethylengruppe oder eine Ethylengruppe ist, y eine ganze Zahl von 1 bis 3 ist,
n die Zahl der positiven Ladungen im Kation und A ein m-wertiges physiologisch verträgliches Anion ist,
zum Färben von Keratinfasern, insbesondere von menschlichen Haaren.

2. Verwendung von Methinfarbstoffen der Formel (I) gemäß Anspruch 1, **dadurch gekennzeichnet, dass** R³ eine Methylgruppe darstellt.

3. Verwendung von Methinfarbstoffen der Formel (I) gemäß einem der Ansprüche 1 oder 2, **dadurch gekennzeichnet, dass** R⁴ und R⁵ Methylgruppen darstellen.

4. Verwendung von Methinfarbstoffen der Formel (I) gemäß einem der Ansprüche 1 bis 3, **dadurch gekennzeichnet, dass** A ein Anion aus der Gruppe Chlorid, Bromid, Jodid, Sulfat, Perchlorat, Tetrachlorozinkat, Tetrafluoroborat oder Tetraphenylborat ist.

5. Haarfärbemittel, **dadurch gekennzeichnet, dass** es Methinfarbstoffe der Formel (I) gemäß einem der Ansprüche 1 bis 4 in einer Menge von 0,01 bis 10 Gew.-%, bezogen auf das gesamte Haarfärbemittel, in einem zum Färben geeigneten Träger enthält.

6. Mittel nach Anspruch 5, **dadurch gekennzeichnet, dass** es frei von Oxidationsfarbstoffvorprodukten ist.

7. Mittel nach Anspruch 6, **dadurch gekennzeichnet, dass** es sich um ein haarfestigendes Mittel handelt.

8. Mittel nach Anspruch 5, **dadurch gekennzeichnet, dass** es mindestens eine Entwicklerkomponente und/oder mindestens eine Kupplerkomponente enthält.

9. Mittel nach Anspruch 8, **dadurch gekennzeichnet, dass** die Entwicklerkomponente ausgewählt ist aus p-Aminophenol, 1-(2'-Hydroxyethyl)-2,5-diaminobenzol, 2,4,5,6-Tetraaminopyrimidin, 2-Hydroxy-4,5,6-triaminopyrimidin, 4-Hydroxy-2,5,6-triaminopyrimidin, 4,5-Diamino-1-(2-hydroxy-ethyl)-pyrazol, p-Toluylendiamin, 2-Aminomethyl-4-amino-phenol, N,N-Bis-(2-hydroxy-ethyl)-p-phenylendiamin, p-Phenylendiamin, 3-Methyt-p-aminophenol und Bis-(2-hydroxy-5-amino-phenyl)-methan.

10. Mittel nach einem der Ansprüche 8 oder 9, **dadurch gekennzeichnet, dass** die Kupplerkomponente ausgewählt ist aus der Gruppe, die von 1-Naphthol, 1,5-, 2,7- und 1,7-Dihydroxynaphthalin, Resorcin, 2,5-Dimethylresorcin, 2,6-Dihydroxy-3,4-diaminopyridin, m-Aminophenol, 5-Amino-2-methylphenol, 3-Methylsulfonylamino-2-methyl-anilin, 3-Amino-2-methylamino-6-methoxy-pyridin, 2,6-Dimethyl-3-aminophenol, 2,4-Diamino-phenoxyethanol, 6-Methyl-1,2,3,4-tetrahydro-chinoxalin, 2-Methyl-4-chlor-5-amino-phenol, 3,4-Methylendioxyanilin, 2-Methyl-resorcin, 5-Methylresorcin, 4-Chlorresorcin, 3,4-Methylendioxyphenol, 2-Amino-3-hydroxypyridin und 2-Chlor-6-methyl-3-aminophenol gebildet wird.

11. Mittel nach einem der Ansprüche 5 bis 10, **dadurch gekennzeichnet, dass** es weiterhin ein anionisches, nichtionogenes oder kationisches Polymeres enthält.

12. Verwendung eines Mittels nach einem der Ansprüche 5 bis 11 zum Färben und Tönen von Haaren.

## Claims

1. Use of methine dyes of the formula (I) 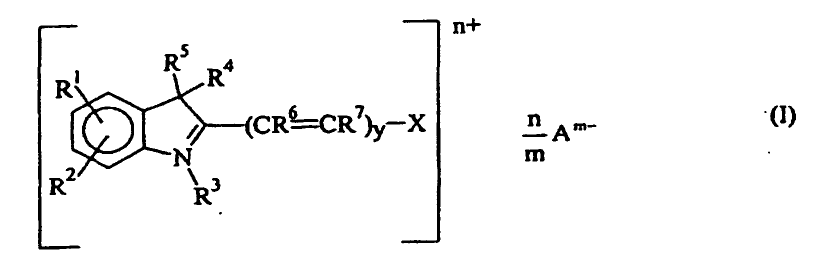 in which R¹ and R², independently of one another, are hydrogen, a C₁- to C₄-alkyl group, a C₁- to C₄-alkoxy group, a hydroxyl group, an amino group or a mono- or di(C₁- to C₄-alkyl)amino group,
R³ is hydrogen, a C₁- to C₄-alkyl group or a C₂- to C₄-hydroxyalkyl group,
R⁴ to R⁷, independently of one another, are hydrogen or a C₁- to C₄-alkyl group and
X is a group of the formulae (II) to (IV) 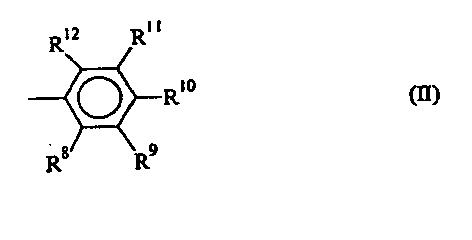 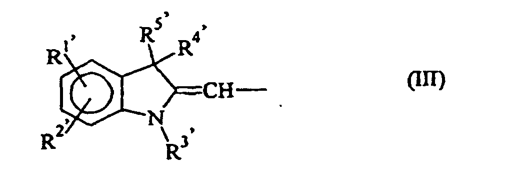 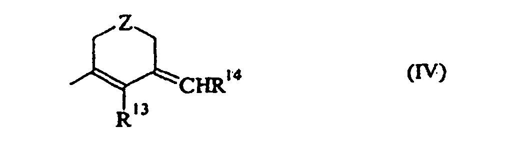 where R^{1'} to R^{5'} are one of the groups specified correspondingly under R¹ to R⁵,
R⁸ and R¹², independently of one another, are hydrogen, a halogen atom, a hydroxyl group, a C₁-to C₄-alkoxy group or a C₂- to C₄-hydroxyalkyl group,
R⁹ and R¹¹ are hydrogen or, in each case together with R¹⁰, form a methylenedioxy group,
R¹⁰ is also a C₁- to C₄-alkoxy group, a C₂- to C₄-hydroxyalkyl group,
a group of the formula (V)
-NR¹⁵R¹⁶ (V)
where R¹⁵ and R¹⁶, independently of one another, are a C₁- to C₄-alkyl group, a C₂- to C₄-hydroxyalkyl group or, together with the nitrogen atom, form a pyrrolidine, piperidine or piperazine ring,
two adjacent substituents R⁸ to R¹² in each case together form a fused benzene ring,
or the substituent according to formula (II) is a 2,3,6,7-tetrahydro-1H,5H-pyrido[3,2,1-ij]quinoline radical,
R¹³ is hydrogen, a halogen atom, a C₁- to C₄-alkyl group, or a C₁- to C₄-alkoxy group,
R¹⁴ is a phenyl or naphthyl radical which is substituted by C₁- to C₄-alkyl groups, C₁- to C₄-alkoxy groups, a hydroxyl group, a carboxyl group or a mono- or di(C₁- to C₄-alkyl)amino group, where the alkyl radicals may also form one or two fused heterocycles with the phenyl or naphthyl radical or one heterocycle with the amine nitrogen, and is pyridyl, quinolyl, furyl, thienyl radicals or the radical (III),
Z is a direct bond, a methylene group, a dimethylmethylene group or an ethylene group, y is an integer from 1 to 3,
n is the number of positive charges in the cation and A is an m-valent physiologically compatible anion,
for dyeing keratin fibres, in particular human hair.

2. Use of methine dyes of the formula (I) according to Claim 1, **characterized in that** R³ is a methyl group.

3. Use of methine dyes of the formula (I) according to one of Claims 1 and 2, **characterized in that** R⁴ and R⁵ are methyl groups.

4. Use of methine dyes of the formula (I) according to one of Claims 1 to 3, **characterized in that** A is an anion from the group chloride, bromide, iodide, sulphate, perchlorate, tetrachlorozincate, tetrafluoroborate or tetraphenylborate.

5. Hair dye, **characterized in that** it comprises methine dyes of the formula (I) according to one of Claims 1 to 4 in an amount of from 0.01 to 10% by weight, based on the total hair dye, in a carrier suitable for the dyeing.

6. Agent according to Claim 5, **characterized in that** it is free from oxidation dye precursors.

7. Agent according to Claim 6, **characterized in that** it is a hair-setting agent.

8. Agent according to Claim 5, **characterized in that** it comprises at least one developer component and/or at least one coupler component.

9. Agent according to Claim 8, **characterized in that** the developer component is chosen from p-aminophenol, 1-(2'-hydroxyethyl)-2,5-diaminobenzene, 2,4,5,6-tetraaminopyrimidine, 2-hydroxy-4,5,6-triaminopyrimidine, 4-hydroxy-2,5,6-triaminopyrimidine, 4,5-diamino-1-(2-hydroxyethyl)pyrazole, p-tolylenediamine, 2-aminomethyl-4-aminophenol, N,N-bis(2-hydroxyethyl)-p-phenylenediamine, p-phenylenediamine, 3-methyl-p-aminophenol and bis(2-hydroxy-5-aminophenyl)methane.

10. Agent according to one of Claims 8 and 9, **characterized in that** the coupler component is chosen from the group which is formed by 1-naphthol, 1,5-, 2,7- and 1,7-dihydroxynaphthalene, resorcinol, 2,5-dimethylresorcinol, 2,6-dihydroxy-3,4-diaminopyridine, m-aminophenol, 5-amino-2-methylphenol, 3-methylsulphonylamino-2-methylaniline, 3-amino-2-methylamino-6-methoxypyridine, 2,6-dimethyl-3-aminophenol, 2,4-diaminophenoxyethanol, 6-methyl-1,2,3,4-tetrahydroquinoxaline, 2-methyl-4-chloro-5-aminophenol, 3,4-methylenedioxyaniline, 2-methylresorcinol, 5-methylresorcinol, 4-chlororesorcinol, 3,4-methylenedioxyphenol, 2-amino-3-hydroxypyridine and 2-chloro-6-methyl-3-aminophenol.

11. Agent according to one of Claims 5 to 10, **characterized in that** it further comprises an anionic, nonionogenic or cationic polymer.

12. Use of an agent according to one of Claims 5 to 11 for dyeing and tinting hair.

## Revendications

1. Utilisation de colorants méthine de formule (I) 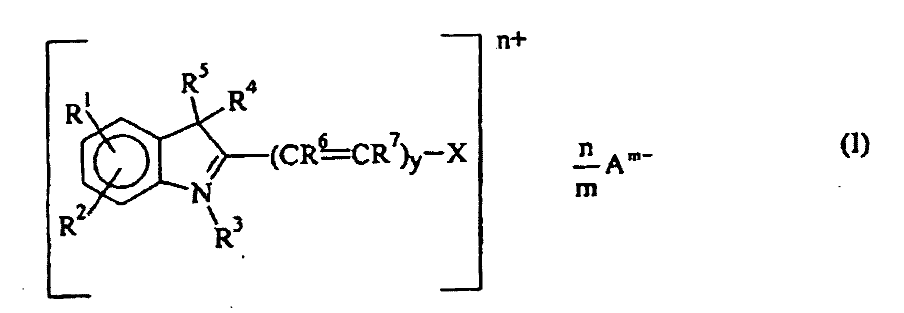 dans laquelle R¹ et R² représentent indépendamment l'un de l'autre un hydrogène, un groupe alkyle en C₁ à C₄, un groupe alcoxy en C₁ à C₄, un groupe hydroxy, un groupe amino ou un groupe mono- respectivement di-(alkyle en C₁ à C₄)-amino,
R³ représente un hydrogène, un groupe alkyle en C₁ à C₄ ou un groupe hydroxyalkyle en C₂ à C₄,
R⁴ à R⁷ représentent indépendamment l'un de l'autre un hydrogène ou un groupe alkyle en C₁ à C₄ et
X représente un groupe de formules (II) à (IV) 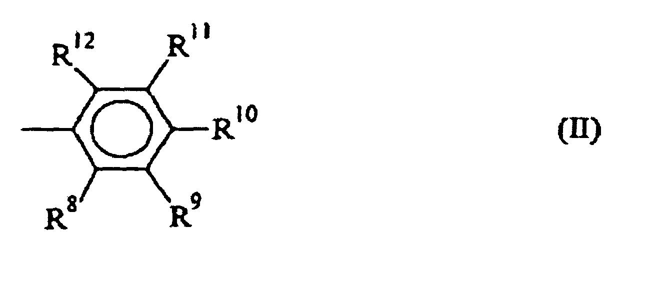 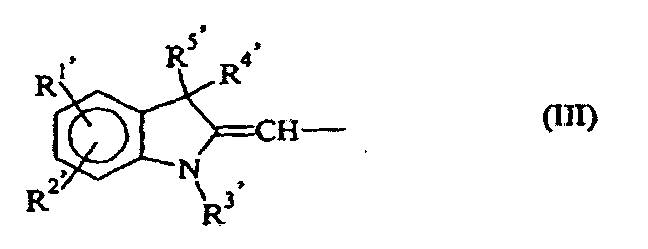 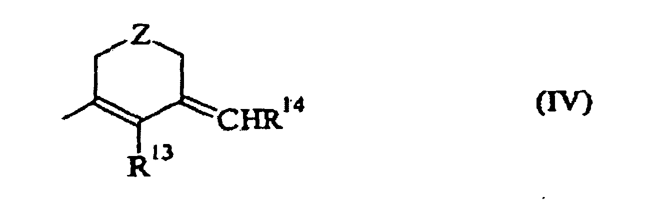 dans lesquelles R^{1'} à R^{5'} représentent l'un des groupes mentionnés de façon correspondante sous R¹ à R⁵, R⁸ et R¹² représentent indépendamment un hydrogène, un atome d'halogène, un groupe hydroxy, un groupe alcoxy en C₁ à C₄ ou un groupe hydroxyalkyle en C₂ à C₄,
R⁹ et R¹¹ représentent l'hydrogène ou forment à chaque fois ensemble avec R¹⁰ un groupe méthylènedioxy,
R¹⁰ représente en outre un groupe alcoxy en C₁ à C₄, un groupe hydroxyalkyle en C₂ à C₄,
un groupe de formule M
-NR¹⁵R¹⁶ (V)
dans laquelle R¹⁵ et R¹⁶ représentent indépendamment l'un de l'autre un groupe alkyle en C₁ à C₄, un groupe hydroxyalkyle en C₂ à C₄ ou forment ensemble avec l'atome d'azote un noyau pyrrolidine, pipéridine ou pipérazine,
deux substituants R⁸ à R¹² voisins forment à chaque fois un noyau benzène condensé
ou le substituant selon la formule (II) est un radical 2,3,6,7-tétrahydro-1 H,5H-pyrido[3,2,1-ij]-quinoléine,
R¹³ représente un hydrogène, un atome d'halogène, un groupe alkyle en C₁ à C₄ ou un groupe alcoxy en C₁ à C₄,
R¹⁴ représente un radical phényle ou naphtyle, qui sont substitués par des groupes alkyle en C₁ à C₄, des groupes alcoxy en C₁ à C₄, un groupe hydroxy, un groupe carboxy ou un groupe mono- respectivement di-(alkyle en C₁ à C₄)-amino, où les radicaux alkyle peuvent également former avec le radical phényle ou naphtyle un ou deux hétérocycles condensés, ou avec l'azote de l'amine un hétérocycle, ainsi que des radicaux pyridyle, quinoléyle, furyle, thiényle ou le radical (III),
Z représente une liaison directe, un groupe méthylène, un groupe diméthylméthylène ou un groupe éthylène, y est un nombre entier allant de 1 à 3,
n est le nombre de charges positives dans le cation et A est un anion physiologiquement acceptable m-valent,
pour la coloration de fibres kératiniques, en particulier de cheveux humains.

2. Utilisation de colorants méthine de formule (I) selon la revendications 1, **caractérisée en ce que** R³ représente un groupe méthyle.

3. Utilisation de colorants méthine de formule (I) selon l'une des revendications 1 ou 2, **caractérisée en ce que** R⁴ et R⁵ représentent des groupes méthyle.

4. Utilisation de colorants méthine de formule (1) selon l'une des revendications 1 à 3, **caractérisée en ce que** A est un anion du groupe formé par le chlorure, le bromure, l'iodure, le sulfate, le perchlorate, le tétrachlorozincate, le tétrafluoroborate ou le tétraphénylborate.

5. Colorant pour cheveux, **caractérisé en ce qu'**il contient des colorants méthine de formule (I) selon l'une des revendications 1 à 4 en une quantité de 0,01 à 10 % en poids, par rapport à l'ensemble du colorant pour cheveux, dans un support approprié pour la coloration.

6. Produit selon la revendication 5, **caractérisé en ce qu'**il est dépourvu de précurseurs de colorants par oxydation.

7. Produit selon la revendication 6, **caractérisé en ce qu'**il s'agit d'un produit fixateur pour cheveux.

8. Produit selon la revendication 5, **caractérisé en ce qu'**il contient au moins un composant de développement et/ou au moins un composant de couplage.

9. Produit selon la revendication 8, **caractérisé en ce que** le composant de développement est choisi parmi le p-aminophénol, le 1-(2'-hydroxyéthyl)-2,5-diaminobenzène, la 2,4,5,6-tétraaminopyrimidine, la 2-hydroxy-4,5,6-triaminopyrimidine, la 4-hydroxy-2,5,6-triaminopyrimidine, le 4,5-diamino-1-(2-hydroxy-éthyl)-pyrazole, la p-toluylènediamine, le 2-aminométhyl-4-amino-phénol, la N,N-bis-(2-hydroxy-éthyl)-p-phénylènediamine, la p-phénylènediamine, le 3-méthyl-p-aminophénol et le bis-(2-hydroxy-5-aminophényl)-méthane.

10. Produit selon l'une des revendications 8 ou 9, **caractérisé en ce que** le composant de couplage est choisi dans le groupe qui est formé par le 1-naphtol, le 1,5-, le 2,7- et le 1,7-dihydroxynaphtalène, la résorcine, la 2,5-diméthylrésorcine, la 2,6-dihydroxy-3,4-diaminopyridine, le m-aminophénol, le 5-amino-2-méthylphénol, la 3-méthylsulfonylamino-2-méthyl-aniline, la 3-amino-2-méthylamino-6-méthoxy-pyridine, le 2,6-diméthyl-3-amino-phénol, le 2,4-diamino-phénoxyéthanol, la 6-méthyl-1,2,3,4-tétrahydro-quinoxaline, le 2-méthyl-4-chloro-5-amino-phénol, la 3,4-méthylènedioxyaniline, la 2-méthyl-résorcine, la 5-méthyl-résorcine, la 4-chloro-résorcine, le 3,4-méthylènedioxyphénol, la 2-amino-3-hydroxypyridine et le 2-chloro-6-méthyl-3-aminophénol.

11. Produit selon l'une des revendications 5 à 10, **caractérisé en ce qu'**il contient en outre un polymère anionique, non ionogène ou cationique.

12. Utilisation d'un produit selon l'une des revendications 5 à 11 pour la coloration et la teinture des cheveux.
